# EUROPEAN PATENT APPLICATION

(11) **EP 1 043 402 A2**
(43) Date of publication of application: **11.10.2000**
(21) Application number: 00302765.3
(22) Date of filing: 31.03.2000
(51) Int. Cl.: C12Q 1/02

(54) **Assays for the determination of pesticidal damage, resistance, and pesticide mode of action**

(30) Priority: 06.04.1999 US 287474
(71) Applicant: American Cyanamid Company, Madison, New Jersey 07940-0874 (US)
(72) Inventor: Singh, Bijay Singh, Robbinsville, New Jersey 08691 (US)
(74) Representative: Walters, Philip Bernard William

(57) **Abstract**

The present invention relates to an assay, and methods for the use thereof, for the assessment and quantification of pesticidal damage to plants, insects or pathogens which comprises an enzyme-deficient microbial culture to which is added the extract of a plant, insect or pathogen in which such damage is suspected. The culture is then allowed a suitable period of growth, and such growth is indicative of the amount of the damage resultant from pesticidal exposure of the plant, insect or pathogen.

## Description

### FIELD OF THE INVENTION

The present invention relates to the assessment of pesticidal damage, pesticide resistance and pesticide mode of action in various agricultural products, especially food crops.

### BACKGROUND OF THE INVENTION

The present invention relates generally to the detection and measurement of enzyme substrates and other intermediates of the pathway, particularly in plants, insects, and pathogens such as fungi.

It has long been known that the inhibition of an enzyme leads to the accumulation of its substrate(s) and intermediates in the pathway. The measurement of these products can provide a quantitative assessment of the extent of the inhibition. However, the conventional methods for measuring the presence of these substrates and intermediates typically involve tedious extractions and purification of the particular substrate or intermediate and measurement with expensive and bulky equipment.

In plants, insects and pathogens, pesticidal damage is frequently the result of enzyme inhibition. Such enzyme inhibition leads to accumulation of intermediates of the pathway. For example, inhibition of acetohydroxyacid synthase (AHAS) by imidazolinone or sulfonylurea herbicides causes the accumulation of 2-ketobutyrate (Shaner and Singh, "Phytotoxicity of acetohydroxyacid synthase inhibitors is not due to accumulation of 2-ketobutyrate and/or 2-aminobutyrate" *Plant Physiol.* 103:1221-1226, **1993,** Singh and Shaner, "Biosynthesis of branched chain amino acids: From test tube to field" *The Plant Cell* 7:935-944, **1995**). Similarly, inhibition of EPSP synthase by glyphosate causes accumulation of shikimate in plants (Amrhein *et al*., "The site of inhibition of the shikimate pathway by glyphosate" *Plant Physiol*., 66:830-834, **1980,** Amrhein *et al*., "Interference of glyphosate with the shikimate pathway" *Proc. Plant Growth Regul. Soc. Am.* 8:99-106, **1981**). Other well-known intermediates that accumulate due to an enzyme inhibition include protoprophyrin IX, due to inhibition of protoporphyrinogen oxidase due to different classes of inhibitors (Becerril and Duke, "Protoporphyrin IX content correlates with activity of photobleaching herbicides", *Plant Physiol*, 90, 1175-1181, **1989**); acetolactate, due to inhibition of acetolactate reductoisomerase by Hoe 704 (Schulz et al. "The herbicidally active experimental compound Hoe 704 is a potent inhibitor of enzyme acetolactate reductiosomerase", FEBS *Letters*, 238, 375-378, **1988**); and ammonia, due to inhibition of glutamine synthetase by bialaphos (Martin et al. "Effect of methionine sulfoximine on the accumulation of ammonia in C3 and C4 leaves", *Plant Physiol*. 71, 177-181, **1983;** Wils, Sauer and Ruhle, "The effect of phosphinothricin on photosynthesis I. Inhibition of photosynthesis and accumulation of ammonia", *Z Naturforsch*. 42c, 263-269, **1986**).

There is a need in the art for an assay which can provide a clear and rapid assessment and quantification of the damage resultant from various pesticide, especially herbicide, products. Especially desirable would be an assay which would not need the support of complicated and expensive laboratory procedures and equipment. Such an assay could also be utilized to specifically identify the particular enzyme which is being inhibited by the pesticide, and to quantify the amount of such inhibition. It is to these aims that the present invention is directed.

The citation of references herein shall not be construed as an admission that such is prior art to the present invention.

### SUMMARY OF THE INVENTION

The present invention is directed to a method of determining damage in plants, insects and pathogens; resultant from exposure to a suspected pesticidal agent which comprises:
a) preparing an extract of a portion of the plant, insect or pathogen;
b) inoculating an enzyme-deficient microbial culture with said extract;
c) allowing the culture to incubate for a standard period of time;
d) incubating a control or standard enzyme-deficient microbial culture; and assessing the amount of growth of the microbe either by comparison to said control or standard in order to assess the type and degree of damage from pesticidal exposure. Further, this invention relates to an assay, or combination of assays, and kits thereof, for use in the method.

In the method of the present invention, the enzyme-deficient microbial strains are used to assess the enzymatic damage to plants, insects or pathogens by a particular pesticidal agent, especially by a herbicide. Using a culture of an enzyme deficient microbe, the ability of the microbe to grow when an extract of a plant, insect or pathogen treated with a pesticide is added provides a measurement of the extent of enzyme inhibition or damage to the organism. Utilizing the same theory, the assays and methods of the present invention can be used to identify crops which have been treated with various pesticides, as well as particular strains of crops and/or weeds and/or plants which are either tolerant or resistant to certain pesticides. Finally, the methods and assays of the present invention can be utilized to identify the mechanism of action of a particular pesticide so that the impact of the pesticide on other plant and animal species can be determined without the need for actual testing of the various species.

It is thus a primary object of the invention to provide a method for the assessment and measurement of the type and extent of herbicidal damage to plants, and particularly to agricultural crops.

A further object of the invention is to provide a method for the assessment and measurement of pesticidal resistance in plants, insects or pathogens and particularly in crops, weeds, or other undesirable plants or pests which are detrimental to commercially important agricultural crops. Such assessment can identify strains of crops which are resistant to the pesticides which are most detrimental to the weeds, insects, or pathogens such as fungi typically encountered in the area, as well as identify particular weed, fungal or insect species which is a particular problem due to their resistance to a particular type of pesticide, especially a herbicide.

Yet a further object of the invention is to provide a method which can be used to identify and assess the mode of action of a particular pesticidal agent. Such identification is particularly important for the assessment of potential damage to other plant and animal species which may come into contact with the particular pesticide.

A further object of the present invention is the assay or kit utilized in the practice of the method of the invention.

A still further object of the invention is to provide an inexpensive, portable assay which can be utilized in the field by relatively unskilled technicians to make a qualitative assessment of pesticidal damage to plant material, especially from a herbicide.

These and other objects of the invention will be better understood by reference to the following drawings, detailed description of the invention, and the Examples.

### BRIEF DESCRIPTION OF THE DRAWINGS

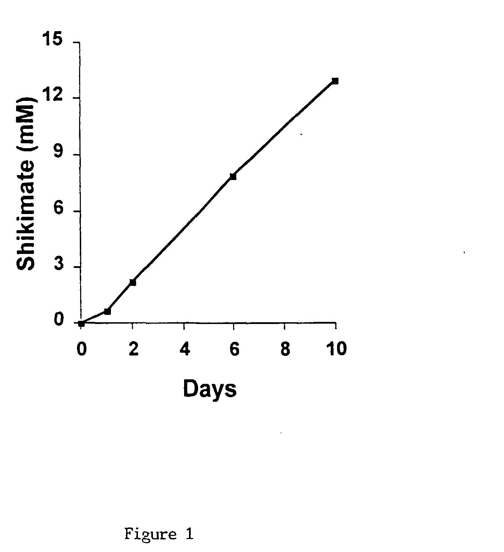

FIGURE 1 is a graph showing the accumulation of shikimate in glyphosate-treated corn plants. The treated plants were spayed with 750 g/Ha rate of glyphosate and the plants were harvested on different days. Extraction of the plants and spectrophotometric analysis thereof is as described in Example 1.

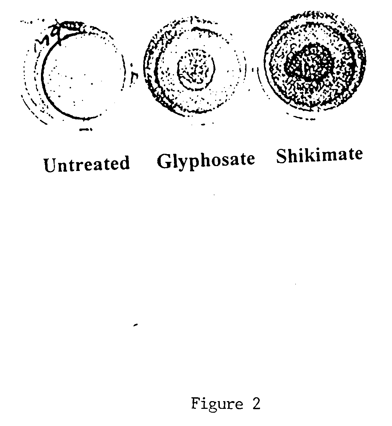

FIGURE 2 is a depiction of the growth of AB2826 strain of *E. coli* on M9 agar medium containing extracts from untreated or glyphosate-treated plants six (6) days after treatment. Pure shikimate (1mM) was used as a positive control.

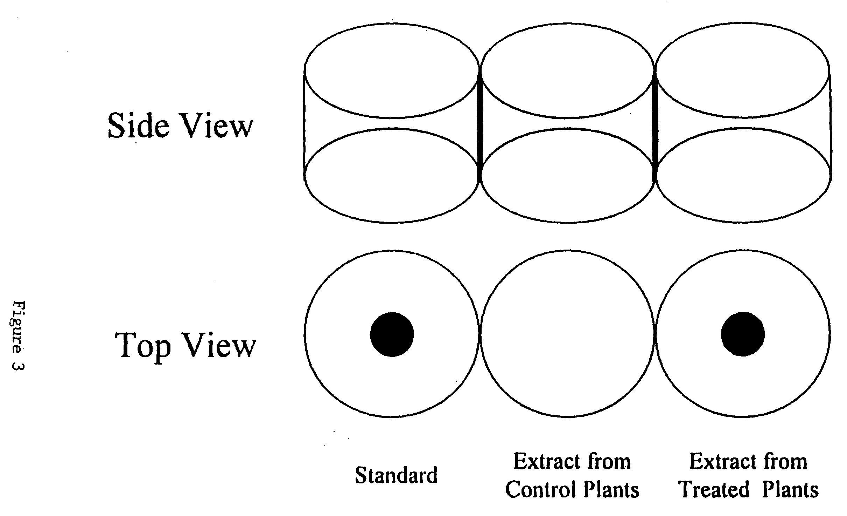

FIGURE 3 is a depiction of a typical apparatus to be utilized in the conduct of the assays of the present invention. This simple system can contain three wells containing M9 agar medium. In the center of these wells is placed a 10 *µ*l aliquot of 1:1000 dilution of an appropriate strain of *E. coli* cells grown overnight. These wells can be stored at 4°C until use. A standard solution of the metabolite (*e.g.* shikimate) will be applied to one well as a positive control and extracts from an untreated and a treated plant will be applied to the other two wells. The plate can then be placed at room temperature or at 37°C for 24 hours to allow bacterial growth. Growth of cells in the well containing extract from a treated plant will indicate the presence of a metabolite that complements the growth of *E. coli*.

### DETAILED DESCRIPTION OF THE INVENTION

The present invention thus relates to methods of determining damage in plants, insects or pathogens resultant from exposure to a suspected pesticidal agent which comprise the preparation of an extract of a portion of the plant; inoculation of an enzyme-deficient microbial culture with said extract; incubation of the culture for a standard period of time to allow growth if such growth is possible and assessment of the amount of growth of the microbe either by comparison to a control or standard in order to assess the type and degree of damage from pesticidal exposure.

In a preferred embodiment, the enzyme-deficient microbial culture is selected from the group consisting of the AB2826 strain of *Escherichia coli (aroB351)* obtained from A.J. Pittard, University of Melbourne, Victoria, Australia. In the typical utilization of the invention, this method may be repeated with different enzyme-deficient microbial cultures in order to identify the general type of pesticide to which the plant has been exposed. In a particularly preferred embodiment, the method of the present invention can be used to identify the herbicide(s) to which plant material is susceptible, or to which it has been exposed.

A variety of cultures can be included on a single culture apparatus in order to enable multiple tests using a single extract, or if desirable, the microbial cultures can each individually be present on a single culture apparatus.

A further embodiment of the present is a method of identifying plant material which is resistant to the effect of a particular class of herbicidal/pesticidal agents which comprises the selection of an enzyme-deficient microbial strain which correlates with the type of enzymatic inhibition exhibited by said class of herbicidal/pesticidal agents; preparation of an extract of a portion of said plant material; inoculation of a culture of said enzyme-deficient microbial strain with said extract; incubation of the culture for a standard period of time; and assessment of the amount of growth of the microbe either by comparison to said control or standard in order to assess the presence and degree of resistance of the plant material to damage from exposure of said class of pesticidal/herbicidal agents. In another preferred embodiment, the class of pesticidal/herbicidal agents is an imidazolinone or sulfonylurea herbicide and the enzyme-deficient microbial strain is deficient in threonine deaminase. Another preferred embodiment is the method wherein the pesticidal/herbicidal agent is glyphosate and the enzyme-deficient microbial strain in deficient in EPSP synthase.

A preferred microbial stain for use in this embodiment is the enzyme-deficient AB2826 strain of *Escherichia coli (aroB351)*, obtained from A.J. Pittard, University of Melbourne, Victoria, Australia, deposited with the American Type Culture Collection (ATCC) on March 16, 1999, and assigned Accession Number ATCC 202209.

Still another embodiment of the present invention involves a method for determining the mode of action of a pesticidal/herbicidal agent which comprises application of the pesticidal/herbicidal agent to plant material wherein the pesticidal/herbicidal activity is observed; preparation of an extract of a portion of said plant material; inoculation of a culture of an enzyme-deficient microbial strain with said extract; incubation of the culture for a standard period of time to allow for growth;
observation of the effects of said extract on said culture in order to determine if the activity of pesticidal/herbicidal is consistent with the enzymatic deficiency of said microbial strain and detection of growth of the culture indicates the presence of pesticidal/herbicidal activity via the inhibition of the enzyme which is deficient in the culture. Typically this method can utilize an enzyme-deficient microbial strain which is deficient in acetohydroxyacid synthase or EPSP synthase.

A still further embodiment of the present invention comprises a method for monitoring the application of a pesticidal/herbicidal agent to an agricultural crop to prevent damage to said crop which comprises sequential and repeated use of the method of the aforesaid methods.

The present invention also includes test kits for assessing and/or quantifying the amount of herbicidal/pesticidal damage to an agricultural crop for use in the various described methods of the present invention. These test kits include one or more enzyme-deficient microbial cultures selected for correlation to the ability of a particular class of pesticidal/herbicidal agents via enzymatic inhibition in plant material; other reagents and equipment, such as those necessary for the preparation of an extract of the plant material; and directions for use of the kit.
In preferred embodiments, these kits can include an enzyme-deficient microbial strain which is deficient in acetohydroxyacid synthase or EPSP synthase. Such kits can find particular utility for the identification of exposure of plant material to imidazolinone or sulfonylurea-type enzymatic inhibition.

Thus, the present invention includes an assay, or combination of assays, which comprise one or more culture mediums containing an enzyme-deficient microbial strain. By adding an extract of a plant treated with a herbicide/pesticide to the culture medium containing the particular enzyme-deficient microbial strain, the enzymatic damage to plants by a particular herbicidal/pesticidal agent can be assessed and quantified. Suitable culture media are dependent upon the particular microbial strain, and such media are well-known in the art. Typical culture media include, but are not limited to M9 agar medium, NZM medium (Sambrook et al. Molecular Cloning, A Laboratory Manual, Cold Spring Harbor Laboratory Press, 1989); SMM and SC medium (Adams et al., Methods in Yeast Genetics: A Labroratory Course Manual, 1998).
The various enzyme-deficient microbial strains utilized in the assays of the present invention are well-known in the art. Particular strains of *Escherichia coli* having utility in the present invention are those such as
*ilv*^{*-*} (MI262) strain: CGSC # 5769 (Genetic Stock Center, Dept. of Biology, Yale University, New Haven, Ct.); lacks acetohydroxyacid synthase activity.
*proB*^{*-*} strain CGSC # 4513; lacks γ-glutamyl kinase activity;
Δ[*his*^{*-*}*gnd*] (WB353) strain: Nagai et al., Structural and functional conservation of histidinol dehydrogenase between plants and microbes, Proc Natl Acad Sci, 88, 4133-4137, 1991; lacks histidinol dehydrogenase activity;
*ilv*^{*-*} (MF2000) strain: M. Freundlich, State University of New York, Stony Brook, New York; lacks acetohydroxyacid synthase activity;
*gln A*^{*-*} (A 318) strain: Miao et al. Ammonia regulated expression of a soybean gene encoding cytoplasmic glutamine synthetase in transgenic Lotus corniculatus. The Plant Cell, 3, 11-22, 1991; lacks glutamine synthetase activity;
Δ*aroG* Δ*aroF* (HE628) strain: Garner and Herrmann, Operator mutations of the *Escherichia coli aroF* gene. J Biol Chem, 260, 3820-3825, 1985; lacks DAHP synthase activity;
*ribB*^{*-*} and *ribA strain*^{*-*}: Katzenmeir, Klonierung und molekularbiologische Charakterisierung des Gens fur GTP-cyclohydrolase I aus *Escherichia coli*, Thesis , Technical University of Munich, 1991; lacks GTP cyclohydrolase II and dihydroxy butanone phosphate synthase activity; and
*ilvA*^{*-*} (CU5125) strain; Pledger and Umbarger, Isoleucine and valine metabolism in *Escherichia coli*, J Bacteriol, 114, 183-194, 1973; lacks threonine deaminase activity.
As stated earlier, the kits of the present invention may be used to measure the pesticidal/herbicidal damage in plant material. Each kit will contain a pre-inoculated culture plate/dish containing a suitable growth media and an area with one or more of the microbial strains, along with solvents and glassware or plasticware to perform the extraction of the plant material and procure an extract for application to the culture/plate dish, hydrochloric acid, acetic acid or buffered solutions with a pH that will minimize the enzymatic activity on the metabolites.
Representative extraction solvents include, but are not limited to, diluted solutions of hydrochloric acid, typically buffered, hydrochloric acid, acetic acid or buffered solutions with a pH that will minimize the enzymatic activity on the metabolites.

Typically, the kit may also contain various apparatus/implements for facilitating the preparation and application of the extracts to the culture plate/dish, such as pipettes and applicators, along with lids/covers to prevent contamination of the culture plate/dish after the extract has been applied thereto. Suitable directions, along with standards, are also typically included, to facilitate the interpretation of the results of the incubation.

The actual apparatus utilized in the performance of the assays of the present invention may, of course, vary according to the particular situation for which the assay is performed. Thus, an assay to be performed in the field may utilize more portable types of supports for the cultures and be of a disposable nature, while assays designed for laboratory use of the present methods will typically utilize glassware and/or apparatus which can be reused and/or recycled.

The system is very simple that uses any standard plate and standard media that is used in routine microbiological procedures. There is no special requirement for individual strains other than the metabolic lesion which is already given in the references for each strain.

A typical system is shown in Figure 3 which contains three wells containing M9 agar medium. In the center of these wells, a 10 µl aliquot of 1:1000 dilution of an appropriate strain of E. coli cells grown overnight is placed. These wells can be stored at 4°C till use. A standard solution of the metabolite (e.g. shikimate) will be applied to one well as a positive control and extracts from an untreated and a treated plant will be applied to the other two wells. The plate can then be placed at room temperature or at 37°C for 24 hours to allow bacterial growth. Growth of cells in the well containing extract from a treated plant will indicate the presence of a metabolite that complements the growth of E. coli.

In accordance with the testing techniques discussed above, one class of such kits will contain at least one culture medium containing the particular microbial strain for use in the method of the present invention. More specifically, a preferred kit may contain, for instance, the AB2826 strain of *E. coli* (*aro*B351) which can be used to detect the use of glyphosate on plant material.

In a preferred embodiment, a sample of plant material is tested with multiple numbers of strains to identify the possible herbicidal/pesticidal agent to which the plant material has been exposed.

The following table illustrates the various strains which can be utilized to detect various type of herbicides.

**TABLE 1**

| **STRAIN** | **HERBICIDE DETECTED** |
|---|---|
| AB 2826 | Glyphosate |
| HE 628 | Glyphosate |
| MI262 | Hoe 704 |
| MI262 | IpOHA (N-hydroxy-N-isopropyloxamate) |
| MF2000 | Hoe 704 |
| MF2000 | IpOHA (N-hydroxy-N-isopropyloxamate) |
| CU5125 | Imidazolinone herbicides |
| CU5125 | Sulfonylurea herbicides |
| CU5125 | Triazolopyrimidine Sulfonamide herbicides |
| CU5125 | Pyrithiobac herbicides |

The present invention will be better understood by reference to the following Examples, which are illustrative of the invention, and are not intended as limiting of the invention. Naturally the foregoing formulations are illustrative and may vary within the skill of the art, and are presented herein in fulfillment of the duty to present the best mode for the practice of the invention.

### EXAMPLE 1

**Plant Material.** Corn plants were grown in an artificial potting mixture in a greenhouse or outside in the field. Plants of different ages were used for the various experiments as described hereinbelow. The actively growing portion of the plant, *i.e.* the bottom portion of the corn shoot, was used to prepare the extracts.
**Microbial strain.** AB2826 strain of *Escherichia coli(aro*B351*)* was obtained from Prof. A. J. Pittard, University of Melbourne, Parkville Victoria, Australia). The cells were maintained in M9 medium in the presence of 1 mM shikimate.
**Herbicide Treatment and Plant Extraction.** Corn seedlings were sprayed with 750 g/ha rate of a commercial formulation of glyphosate. Plant material was ground in liquid nitrogen and then further ground in 0.25 N HCl in 1:1 (weight of tissue/volume of extraction buffer) ratio. The extract was centrifuged at 25,000 g for 15 minutes. The supernatant was collected and directly used for the spectrophotometric assay or sterilized by passing through a 0.22µ syringe filter and used for the assay using *E. coli.*
**Spectrophotometric Determination of Shikimate.** Shikimate was determined according to a modification of the method of Gaitonde and Gordon, "A microchemical method for the detection and determination of shikimic acid" *J. Biol. Chem.,* 230:1043-1050, **1958.** An aliquot of the test sample (10-50 µ1) was mixed with 0.5 ml of a 1 % solution of periodic acid to oxidize shikimic acid. After three (3) hours, 0.5 ml of 1N NaOH was added, mixed, and 0.3 ml of 0.1 M glycine was immediately added. The solution was thoroughly mixed and the optical density at 380 nm was immediately measured.
**Determination of Shikimate Using *E. Coli.*** The AB2826 strain of *E. coli* was grown overnight in M63 medium containing 1 mM shikimate. The cells were collected by centrifugation and the medium was discarded. The cells were washed twice with distilled water by vortexing and centrifugation. The cells were then diluted 1000-fold from their original density after overnight culture. An aliquot of these cells (10µl) was placed on an agar plate containing M9 medium and different amounts of extract from untreated and treated plants.

Samples from untreated and glyphosate treated plants were harvested at different times and analyzed for the levels of shikimate in them. The untreated plants had negligible amounts of shikimate in them as shown in the graph of **Figure 1**. In contrast, the concentration of shikimate increased with increasing time following glyphosate treatment.

The AB2826 strain of *E. Coli* did not grow on M9 agar medium containing extracts prepared from untreated plants. Consistent with the spectrophotometric assay, this result indicates that the untreated plants have extremely low levels of shikimate. In contrast, as low as 1 µl of extract prepared from glyphosate treated plants allowed growth of the AB2826 strain of *E. coli.* This result thus confirms the results from the spectrophotometric assay that glyphosate treated plants contain high levels of shikimate which can be utilized as an indicator of glyphosate exposure.

### EXAMPLE 2

Using particular strains of *E. coli* or other microbes that are deficient in different enzymes of different pathways, various metabolites which are the result of enzymatic inhibition can be similarly detected. For instance, corn which is glyphosate-resistant or glyphosate-tolerant will not support the growth of AB2826 strain of *E. coli,* and thus show low levels of shikimate The use of a culture assay avoids the necessary spectrophotometric assay of shikimate for this determination.

To determine the resistance of a particular agricultural crop to a particular type of herbicide which is known to inhibit a certain enzyme, the extract of the plant material can be assayed to determine if it allows the growth of the corresponding enzyme-deficient microbe. If the microbial culture shows growth, then the crop will be sensitive to a herbicide/pesticide with the particular enzyme-inhibiting properties.

Further, the particular type of pesticide to which plant material has been exposed can thus be determined by placing a group of enzyme-deficient strains on a single culture plate, and incubating them with an extract of the plant material under test. By knowing which type of pesticide will allow growth of a particular strain (by prior testing of known pesticides), the nature and/or identity of an unknown pesticide to which the plant was exposed can be determined.

This invention may be embodied in other forms or carried out in other ways without departing from the spirit or essential characteristics thereof. The present disclosure is therefore to be considered as in all respects illustrative and not restrictive, the scope of the invention being indicated by the appended Claims, and all changes which come within the meaning and range of equivalency are intended to be embraced therein

## Claims

1. A method of determining damage in plants, insects or pathogens resultant from exposure to a suspected pesticidal agent which comprises:
a) preparing an extract of a portion of the plant, insect or pathogen;
b) inoculating an enzyme-deficient microbial culture with said extract;
c) allowing the culture to incubate for a standard period of time;
d) incubating a control or standard enzyme-deficient microbial culture; and
e) assessing the amount of growth of the microbe either by comparison to said control or standard in order to assess the type and degree of damage from pesticidal exposure.

2. The method according to Claim 1 wherein the enzyme-deficient microbial culture is an *E. coli* strain selected from the group consisting of the *ilv*-(MI262) strain: CGSC #5769; *pro B*^{*-*} *strain* CGSC# 4513; Δ[*his-gnd*] (WB353) strain; *ilv*-(MF2000) strain; *gln A*^{*-*} (A 318) strain; Δ*aroG* Δ*aroF* (HE628) strain; *rib B*^{*-*} and *ribA* strain; *ilvA*^{*-*} (CU5125) strain.

3. The method according to Claim 1 wherein the method is repeated with two or more different enzyme-deficient microbial cultures in order to identify the general type of pesticide to which the plant has been exposed.

4. The method according to Claim 1 wherein the method includes a variety of cultures on a single culture apparatus in order to enable multiple tests.

5. A method of identifying plant material which is resistant to the effect of a particular class of pesticidal agents which comprises:
a) selecting an enzyme-deficient microbial strain which correlates with the type of type of enzymatic inhibition exhibited by said class of pesticidal agents;
b) preparing an extract of a portion of said plant material;
c) inoculating a culture of said enzyme-deficient microbial strain with said extract;
d) allowing the culture to incubate for a standard period of time;
e) incubating a control or standard enzyme-deficient microbial culture; and
f) assessing the amount of growth of the microbe either by comparison to said control or standard in order to assess the presence and degree of resistance of the plant material to damage from exposure said class of pesticidal/herbicidal agents.

6. A method according to Claim 5 wherein the class of pesticidal agents is an imidazolinone or sulfonylurea herbicide and the enzyme-deficient microbial strain is deficient in threonine deaminase.

7. A method according to Claim 6 wherein the enzyme-deficient strain of *E. coli* is CU5125.

8. A method according to Claim 5 wherein the pesticidal agent is glyphosate and the enzyme-deficient microbial strain is an *E. coli* strain in deficient in EPSP synthase.

9. A method according to Claim 8 wherein the enzyme-deficient strain is the AB2826 strain of *Escherichia coli (aroB351),* deposited with the American Type Culture Collection (ATCC) on March 16, 1999, and assigned Accession Number 202209.

10. A method for determining the mode of action of a pesticidal agent which comprises:
a) applying said pesticidal agent to plant, insect or pathogen material wherein the pesticidal activity is observed;
b) preparing an extract of a growing portion of said plant material;
c) inoculating a culture of an enzyme-deficient microbial strain with said extract;
d) allowing the culture to incubate for a standard period of time;
observing the effects of said extract on said culture in order to determine if the activity of pesticidal is consistent with the enzymatic deficiency of said microbial strain and detection of growth of the culture indicates the presence of pesticidal/herbicidal activity via the inhibition of the enzyme which is deficient in said culture.

11. A method according to Claim 10 wherein the enzyme-deficient microbial strain is an *E. coli* strain deficient in threonine deaminase.

12. A method according to Claim 10 wherein the enzyme deficient microbial strain is an *E. coli* strain deficient in EPSP synthase.

13. A method for monitoring the application of a pesticidal agent to an agricultural crop to prevent damage to said crop which comprises sequential and repeated use of the method of Claim 1.

14. A test kit for assessing and/or quantifying the amount of pesticidal damage to an agricultural crop which comprises;
a) an enzyme-deficient microbial culture selected for correlation to the ability of a particular class of pesticidal agents via enzymatic inhibition in plant material;
b) other reagents; and
c) directions for use of the kit.

15. The test kit of Claim 14, wherein the enzyme-deficient microbial strain is an *E. coli* strain deficient in threonine deaminase.

16. The test kit of Claim 14 wherein the enzyme-deficient microbial strain is an *E. coli* strain deficient in EPSP synthase.

17. A test kit of Claim 14 for detection of imidazolinone or sulfonylurea-type enzymatic inhibition.
